(19) Europäisches Patentamt
European Patent Office
Office européen des brevets

(11) Veröffentlichungsnummer: **0 302 132 B1**

# (12) EUROPÄISCHE PATENTSCHRIFT

(45) Veröffentlichungstag der Patentschrift: **03.04.91**

(51) Int. Cl.5: **C07D 487/04**, B01J 8/08, //A61K31:505,(C07D487/04, 249:00,239:00)

(21) Anmeldenummer: **87111363.5**

(22) Anmeldetag: **06.08.87**

Die Akte enthält technische Angaben, die nach dem Eingang der Anmeldung eingereicht wurden und die nicht in dieser Patentschrift enthalten sind.

## (54) Verfahren und Vorrichtung zur Herstellung von 5-Methyl-7-diethylamino-s-triazolo(1,5-a)-pyrimidin.

(43) Veröffentlichungstag der Anmeldung:
**08.02.89 Patentblatt 89/06**

(45) Bekanntmachung des Hinweises auf die Patenterteilung:
**03.04.91 Patentblatt 91/14**

(84) Benannte Vertragsstaaten:
**AT BE CH DE ES FR GB IT LI LU NL SE**

(56) Entgegenhaltungen:
**DD-A- 55 956**
**DD-A- 70 311**
**DD-A- 155 068**

(73) Patentinhaber: **Deutsche Hydrierwerke GmbH Rodleben**

**O-4530 Rosslau (Elbe)(DE)**

(72) Erfinder: **Völkner, Heinz, Dipl.-Ing.**
**August-Bebel-Strasse 54**
**O-4500 Dessau(DE)**
Erfinder: **Eidner, Hans-Joachim, Dipl.-Ing.**
**Ahrenshoober Strasse 49**
**O-1093 Berlin(DE)**

(74) Vertreter: **Hoffmann, Klaus, Dr. rer. nat. et al**
**Hoffmann . Eitle & Partner Patentanwälte**
**Postfach 81 04 20 Arabellastrasse 4**
**W-8000 München 81(DE)**

**EP 0 302 132 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren und eine Vorrichtung zur Herstellung von 5-Methyl-7-diethylamino-s-triazelo(1,5-a)pyrimidin gemäß Formel (I).

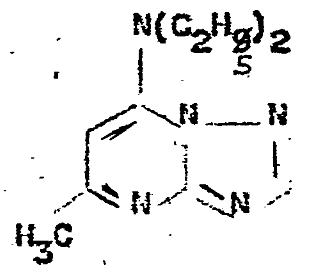

Die Verbindung (I) ist der Wirkstoff eines Arzneimittels, das zur Behandlung von Herzkreislauferkrankungen eingesetzt wird.

Das bisher bekannte Verfahren zur Herstellung der Verßindung (I) ist in den DD-EB 55 956 und 70 311 beschrieben. Es liegt die zur Herstellung eines Pharmazeutika übliche diskontinuierliche Verfahrensweise eines Laborverfahrens zugrunde.
Nachteile dieses Verfahrens sind lange Reaktionszeiten bei der Umsetzung von 5-Methyl-7-hydroxy-s-triazolo(1,5-a) pyrimidin (II) mit Phosphoroxidchlorid (IV) zu 5-Methyl-7-chlor-s-triazolo(1,5-a)-pyrimidin (III), die zur verstärkten Bildung von Nebenprodukten führen, die schwierige Handhabung des entstandenen Zwischenproduktes bei der notwendigen Zersetzung des überschüssigen Reaktionsmittels (IV) und die dabei zu beobachtende hohe Hydrolyseanfälligkeit der Verbindung (III) bei der über 20 °C liegenden Zersetzungstemperatur. Bei der Aminierung der Verbindung (III) mit Diethylamin entstehen neben der gewünschten Verbindung (I) auch die in DD-EB 155 068 beschriebenen unerwünschten Nebenverbindungen
[-7-Diethylamino-s-triazolo(1,5-a)pyrimidinyl-(5)]-
[bis (5-methyl-s-triazolo(1,5-a)pyrimidinyl-(7)]-methan und
[7-Diethylamino-s-triazolo)1,5-a)pyrimidinyl-(5)]-
[5-methyl-s-triazolo(1,5-a)pyrimidinyl-(7)]-methan
als Folge einer langen Reaktionszeit in der Chlorierung, wodurch die Ausbeute negativ beeinflußt wird. Nachteilig wirkt sich auch die relativ geringe Produktionskapazität des geübten diskontinuierlichen Verfahrens aus.

Folglich ist es das Ziel der vorliegenden Erfindung, ein Verfahren und eine Vorrichtung zur Herstellung der Verbindung (I) zu finden, das mit technisch einfachen Mitteln die kompliziert ablaufende Reaktion des Feststoffes (II) mit dem Chlorierungsmittel (IV) ermöglicht, das kurze Reaktionszeiten und damit eine erhöhte Ausbeute ergibt, eine einfache Weiterverarbeitung des Zwischenproduktes (III) ermöglicht und eine Produktion größerer Mengen im technisch durchführbaren Maßstab erlaubt.

Erfindungsgemäß wird diese Aufgabe gelöst, indem die besonderen Bedingungen für ein kontinuierliches Herstellungsverfahren gefunden wurden. Dazu werden die Reaktionskomponenten (II) und (IV) kontinuierlich mit separaten Dosierorganen zwangsweise einem waagerecht angeordneten, unter Normaldruck arbeitenden Reaktionsrohr zugeführt, dort mit Hilfe einer Förderspirale vermsicht, durch intensive indirekte Wärmezufuhr auf Siedetemperatur des Chlorierungsmittels erhitzt und durch anschließende aufeinander abgestimmte Wärmezu- und -abfuhr, die ein ständiges Sieden und Kondensieren des Chlorierungsmittels ermöglicht, zur vollständigen Reaktion gebracht.
Zum Zwecke der aufeinander abgestimmten Wärmezu- und -abfuhr wird nur die untere Seite des waagerechten Reaktionsrohres indirekt beheizt, während die obere Seite unverleidet als Luftkondensator wirkt. Die erfindungsgemäße Vorrichtung ermöglicht Reaktionszeiten von ca. 20 Minuten.
Im Gegensatz dazu werden beim diskontinuierlichen Verfahren Reaktionszeiten von ca. 4 Stunden benötigt. Die entstandene Schmelze läßt sich ohne Schwierigkeiten und ohne Abkühlung einer kontinuierlichen Zersetzung des überschüssigen Chlorierungsmittels und der durch Reaktion enstandenen Phosphorchloride mit Wasser zuführen. Die mit hoher Exothermie ablaufende Zersetzungsreaktion läßt sich durch die definierte Vermischung bei gleichzeitiger Kühlung im kontinuierlichen Betrieb unter Vermeidung von örtlichen Überhitzungen sicher beherrschen. Eine Verwendung von Suspendierungsmitteln beim Transport der Schmelze entfällt. Die sich anschließenden Verfahrensschritte Aminierung, Freisetzung des Diethylamins aus Diethylaminhydrochlorid, Phasentrennung und destillative Befreiung der Reaktionsmischung vom überschüssigen Diethylamin und dem zur besseren Phasentrennung zugesetzten Toluen, bei gleichzeitiger Rückführung derselben, lassen sich durch die kontinuierliche Gestaltung der Chlorrierung der Verbindung (II) zu (III) nun ebenfalls ohne Schwierigkeiten kontinuierlich beherrschen.
Die Vorteile des erfindungsgemäßen Verfahrens und der dazugehörenden Vorrichtung lassen sich wie folgt zusammenfassen:

- Verringerung der Reaktionszeiten von 4 Stunden auf 20 Minuten
- dadurch Erhöhung der Ausbeaute auf 98 % infolge Zuruckdrängung der Konkurrenzreaktion und Vermeidung der Hydrolyse der Verbindung III
- Ermöglichung größerer Mengenumsätze
- Reaktionsablauf in einer technisch einfacheren Vorrichtung
- insgesamt größere Produktivität mit ökonomisch günstigeren Ergebnissen

Ausführungsbeispiel

10 kg der Verbindung (II) werden stündlich über eine Eingabevorrichtung mit einer Dosierschnecke (1) einem Reaktionsrohr (3) zugeführt, in welches gleichzeitig 13 kg/h der Verbindung (IV) mit einer Dosierpumpe gefördert werden. Beide Komponenten werden mechanisch unter Normalbedingungen mit Hilfe einer Förderspirale (2) vermischt und in eine Heizstrecke transportiert, in der das Gemisch auf Siedetemperatur der Verbindung (IV) erhitzt wird. Im anschließenden Reaktionsrohr (3) wird die Reaktion unter ständigem Sieden der Verbindung (IV) zu Ende geführt. Das Reaktionsrohr (3), das nur auf der unteren Seite indirekt beheizt wird, ist so bemessen, daß eine Verweilzeit von ca. 20 Minuten erreicht wird. Die entstandenen Dämpfe der Verbindung (IV) werden an der luftgekühlten Rohroberseite kondensiert und somit unmittelbar der Reaktionsmischung wieder zugeführt. Das Ende des Reaktionsrohres schließt ein Stausystem (4, 5) ab, dessen erster Stau (4) das Austreten von noch nicht umgesetzter Verbindung (II) verhindert. Der zweite Stau (5) reguliert den Füllstand im Reaktionsrohr und bestimmt die Verweilzeit bei gegebenem Durchsatz. Die während der Reaktion entstandene Schmelze fließt kontinuierlich den ebenfalls kontinuierlich betriebenen nachfolgenden Stufen zu. In diesen werden die überschüssigen Reaktionsprodukte mit Natronlauge neutralisiert und die entstandene Verbindung (III) mit Diethylamin aminiert. Die erhaltene Reaktionsmischung wird mit Natronlauge versetzt und in Schichten getrennt. Die den Hauptteil der Verbindung (I) enthaltene Oberschicht wird durch kontinuierliche Wasserdampfdestillation vom überschüssigen Diethylamin befreit. Durch das gemäß DD-EB 155 068 beschriebene Verfahren zur Reindarstellung erhält man 11,5 kg/h (84 % der Theorie) an 5-Methyl-7-diethylamino-s-triazolo(1,5-a)-pyrimidin.

Aufstellung der verwendeten Bezugszeichen

| | |
|---|---|
| 1 | - Dosierschnecke |
| 2 | - Förderspirale |
| 3 | - Reaktionsrohr |
| 4 | - erster Stau |
| 5 | - zweiter Stau |
| II | - Verbindung II - (5-Methyl-7-hydroxy-s-triazolo(1,5-a)pyrimidin |
| III | - Reaktionsprodukt III (5-Methyl-7-chlor-s-triazolo(1,5-a)pyrimidin |
| IV | - Verbindung IV (Phosphoroxidchlorid) |

## Ansprüche

1. Verfahren zur Herstellung von 5-Methyl-7-diethylamino-s-triazolo(1,5-a)pyrimidin (I), dadurch gekennzeichnet, daß das verfahren kontinuierlich gestaltet wird, indem die Komponenten Phosphoroxidchlorid und 5-Methyl-7-hydroxy-s-triazolo(1,5-a)pyrimidin kontinuierlich vermischt, mittels intensiv indirekter Wärmezufuhr auf Siedetemperatur des Phosphoroxidchlorids erhitzt, durch aufeinander abgestimmte Wärmezu- und -abfuhr unter ständigem Sieden des Phosphoroxidchlorids zur vollständigen Reaktion gebracht werden und anschließend die verfahrensschritte Aminierung, Freisetzung des Diethylamins aus Diethylaminhydrochlorid, Phasentrennung und destillative Befreiung der Reaktionsmischung von überschüssigem Diethylamin und dem zur besseren Phasentrennung zugesetzten Toluen bei gleichzeitiger Rückführung derselben in bekannter Weise nun ebenfalls kontinuierlich durchgeführt werden können.

2. Vorrichtung zur kontinuierlichen Herstellung der Verbindung (I) gemäß Anspruch 1, dadurch gekennzeichnet, daß sie aus zwei Teilen besteht, deren erster Teil, bestehend aus einem Doppelmantelrohr mit wandgängiger Förderspirale, die mechanische Vermischung des Feststoffes und der Flüssigkeit unter intensiver Wärmezufuhr ermöglicht und deren zweiter Teil, bestehend aus einem waagerecht angeordneten Reaktionsrohr, das nur auf der Unterseite beheizt wird und durch ein Stausystem abgeschlossen ist, die gleichzeitige Wärmezufuhr und Wärmeabfuhr zur Erzielung einer Reaktion unter ständigem Sieden und Rückfluß der Flüssigkeit gestattet.

## Claims

1. Process for the production of 5-methyl-7-diethylamino-s-triazolo[1,5-a]pyrimidine (I), characterized in that the process is arranged continuously in that the components phosphoryl chloride and 5-methyl-7-hydroxy-s-triazolo[1,5-a]pyrimidine are continuously mixed, heated by means of intensive indirect heat supply at the boiling point of the phosphoryl chloride, and completely reacted by mutually adapted heat supply and removal with continuous boiling of the phosphoryl chloride, and subsequently the process steps of amination, liberation of diethylamine from diethylamine hydrochloride, phase separation and freeing the reaction mixture by distillation

from excess diethylamine and the toluene added to improve the phase separation together with their simultaneous recycle in known manner can now likewise be carried out continuously.

2. Apparatus for the continuous production of the compound (I) according to claim 1, characterized in that it consists of two sections, the first section of which, consisting of a double-shell tube with a spiral conveyer reaching the wall enables the mechanical mixing of the solid and the liquid with intensive heat supply, and the second section of which, consisting of a horizontal reaction tube which is heated only on its underside and closed by a barrier system, permits simultaneous heat supply and heat removal for the realization of a reaction with continuous boiling and reflux of the liquid.

## Revendications

1. Procédé pour la préparation du 5-méthyl-7-diéthylamino-s-triazolo(1,5-a)pyrimidine (I) caractérisé en ce que le procédé est mis en oeuvre en continu, au cours duquel les composants oxychlorure de phosphore et 5-méthyl-7-hydroxy-s-triazolo(1,5-a)-pyrimidine sont mélangés en continu, chauffés par un apport indirect et intensif de chaleur à la température d'ébullition de l'oxychlorure de phosphore, portés à réaction complète grâce à un apport et une extraction de chaleur adaptés l'un en fonction de l'autre avec une ébullition continuelle de l'oxychlorure de phosphore, les étapes suivantes du processus, à savoir l'amination, le dégagement de la diéthylamine du chlorure de diéthylamine, la séparation des phases et la libération par distillation du mélange réactionnel de l'excédent de diéthylamine et du toluène ajouté pour améliorer la séparation des phases, avec recyclage simultané, pouvant être exécutées également en continu de manière connue.

2. Dispositif de préparation en continu du composé (I) selon la revendication 1, caractérisé en ce qu'il est composé de deux parties dont la première constituée d'un tube à double enveloppe avec spirale de transport, jusqu' à la paroi permet le mélange mécanique de la matière solide et du liquide sous apport intensif de chaleur, et dont la deuxième partie constituée d'un tube de réaction disposé horizontalement, chauffé uniquement sur son côté inférieur et fermé par un système de retenue, permet l'apport et l'extraction simultanés de chaleur afin d'obtenir une réaction avec ébullition continuelle et recyclage du liquide.

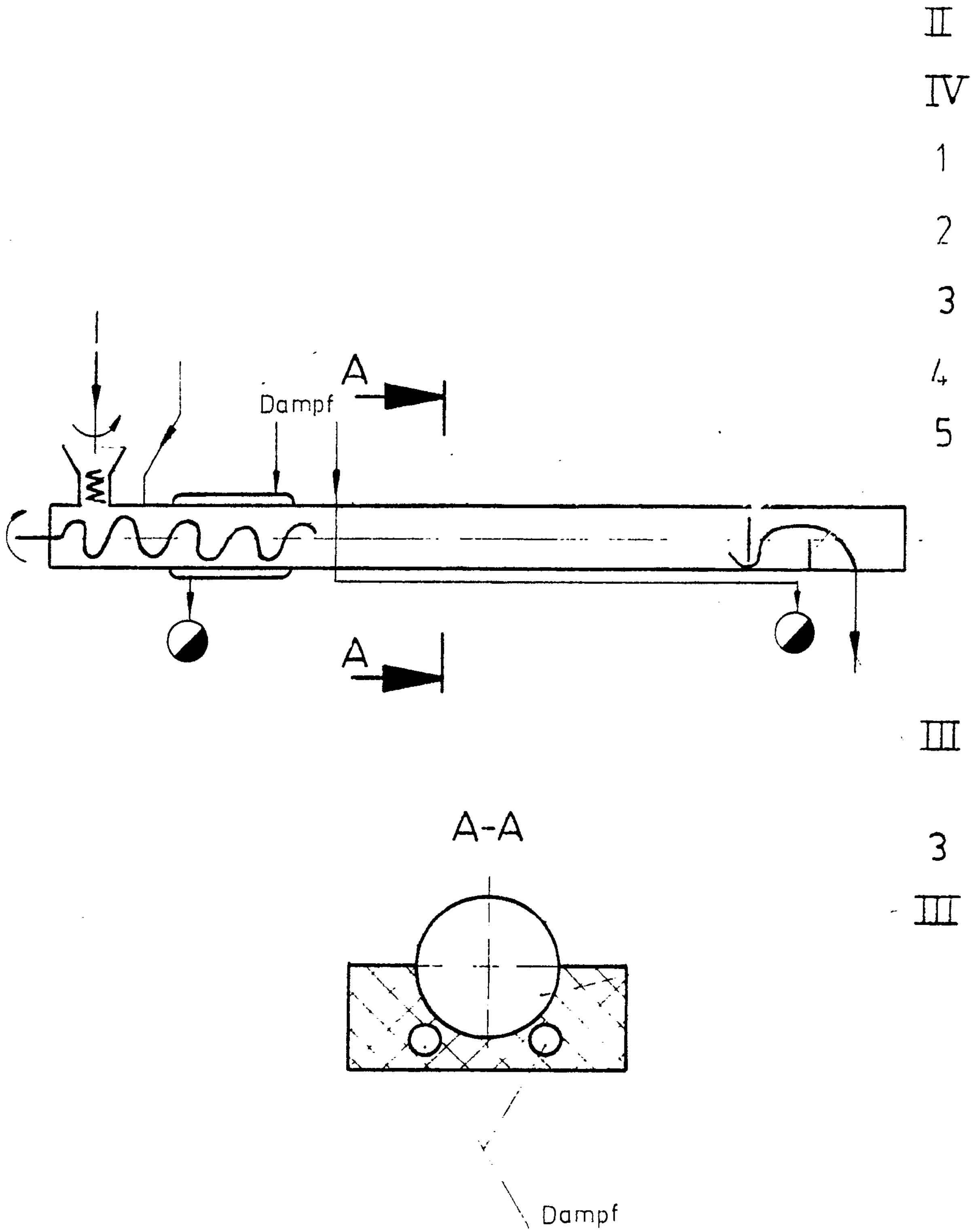


Figur 1